# EUROPEAN PATENT APPLICATION

(11) **EP 4 814 968 A1**
(43) Date of publication of application: **30.09.2026**
(21) Application number: 24912023.9
(22) Date of filing: 05.11.2024
(51) Int. Cl.: G16B 15/00, G16B 40/20

(54) **PHARMACEUTICAL PRODUCT DEVELOPMENT ASSISTANCE DEVICE, OPERATION METHOD FOR PHARMACEUTICAL PRODUCT DEVELOPMENT ASSISTANCE DEVICE, AND OPERATION PROGRAM FOR PHARMACEUTICAL PRODUCT DEVELOPMENT ASSISTANCE DEVICE**

(30) Priority: 25.12.2023 JP 2023218333
(71) Applicant: FUJIFILM Corporation, Tokyo 106-8620 (JP)
(72) Inventor: KATO, Noriji, Ashigarakami-gun, Kanagawa 258-8577 (JP)
(74) Representative: Hoffmann Eitle
(86) International application number: PCT/JP2024/039307
(87) International publication number: WO 2025/142130

(57) **Abstract**

A pharmaceutical product development support device includes a processor, in which the processor acquires target sequence information representing a sequence of amino acids constituting an amino acid-derived substance contained in a target pharmaceutical product, converts the target sequence information into target vector data reflecting a three-dimensional structure of the amino acids, derives target formulation information representing a formulation of a storage solution suitable for the target pharmaceutical product based on the target vector data, and presents the target formulation information to a user.

## Description

### BACKGROUND OF THE INVENTION

### Technical Field

A technology of the present disclosure relates to a pharmaceutical product development support device, an operation method of a pharmaceutical product development support device, and an operation program of a pharmaceutical product development support device.

### Related Art

In recent years, pharmaceutical products such as biopharmaceutical products, peptide pharmaceutical products, and nucleic acid pharmaceutical products have attracted attention due to high drug efficacy and low side effects. As an example, a biopharmaceutical product contains a protein such as interferon or an antibody as an active ingredient. The biopharmaceutical product is preserved in a storage solution. It is important to make a formulation of the storage solution (also referred to as formulation prescription) suitable for the biopharmaceutical product in order to stably maintain a quality of the biopharmaceutical product. Here, the storage solution is composed of a base buffer solution and additives such as a salt, a sugar, an amino acid, and a surfactant. The formulation of the storage solution is, for example, a type of the buffer solution and presence or absence of a salt, a sugar, an amino acid, and a surfactant.

In the related art, in a case of determining the formulation of the storage solution, a plurality of types of storage solutions have been prepared by changing a combination of the type of the buffer solution and the presence or absence of a salt, a sugar, an amino acid, and a surfactant, and storage stability of each storage solution has been confirmed by an actual test. However, it takes a lot of time and effort to perform such an operation. Therefore, for example, WO2019/070517A proposes a technique of predicting a formulation of a storage solution suitable for a biopharmaceutical product based on physical property values of a protein that is an active ingredient of the biopharmaceutical product.

Amino acids constituting a protein have a complex three-dimensional structure (a secondary structure, a tertiary structure, and a quaternary structure), and the three-dimensional structure varies depending on the protein. Even in a case of proteins having substantially the same amino acid sequence (primary structure), the three-dimensional structures are often completely different. Such a difference in three-dimensional structure also affects a formulation of a suitable storage solution. However, in WO2019/070517A, a three-dimensional structure of an amino acid is not taken into consideration. Therefore, there is a possibility that prediction accuracy of the formulation of the storage solution is not sufficient.

### SUMMARY

One embodiment according to the technology of the present disclosure provides a pharmaceutical product development support device, an operation method of a pharmaceutical product development support device, and an operation program of a pharmaceutical product development support device, which can improve prediction accuracy of a formulation of a storage solution.

A pharmaceutical product development support device according to the present disclosure comprises a processor, in which the processor is configured to: acquire target sequence information representing a sequence of amino acids constituting a substance that is amino acid-derived and contained in a target pharmaceutical product; convert the target sequence information into target vector data reflecting a three-dimensional structure of the amino acids; derive target formulation information representing a formulation of a storage solution suitable for the target pharmaceutical product based on the target vector data; and present the target formulation information to a user.

It is preferable that the processor is configured to convert the target sequence information into the target vector data by using a language model that treats the target sequence information as a sentence in natural language processing.

It is preferable that the target formulation information is vector data representing a type of a buffer solution that is a base of the storage solution suitable for the target pharmaceutical product, and presence or absence of a salt, a sugar, an amino acid, and a surfactant contained in the storage solution suitable for the target pharmaceutical product.

It is preferable that the processor is configured to derive the target formulation information by using a machine learning model that is trained by using training data composed of a set of reference vector data converted from reference sequence information representing a sequence of amino acids constituting a substance that is amino acid-derived and contained in a reference pharmaceutical product different from the target pharmaceutical product, and reference formulation information representing a formulation of a storage solution suitable for the reference pharmaceutical product.

It is preferable that the reference formulation information is clustered into one of a plurality of clusters, the machine learning model outputs one cluster of the plurality of clusters in response to input of the target vector data, and the processor is configured to derive, as the target formulation information, the reference formulation information belonging to the cluster output by the machine learning model.

It is preferable that the processor is configured to derive the target formulation information based on a frequency of appearance of the reference formulation information belonging to the cluster output by the machine learning model.

It is preferable that the processor is configured to receive designation of a derivation condition of the target formulation information by the user, and derive the target formulation information based on the derivation condition.

It is preferable that the derivation condition is the number of pieces of the target formulation information to be derived.

It is preferable that the derivation condition is at least one of a type of a buffer solution that is a base of the storage solution or presence or absence of an additive contained in the storage solution.

It is preferable that the reference formulation information is vector data representing a type of a buffer solution that is a base of the storage solution suitable for the reference pharmaceutical product, and presence or absence of a salt, a sugar, an amino acid, and a surfactant contained in the storage solution suitable for the reference pharmaceutical product.

It is preferable that the substance is a protein.

It is preferable that the protein is an antibody.

An operation method of a pharmaceutical product development support device according to the present disclosure comprises: acquiring target sequence information representing a sequence of amino acids constituting a substance that is amino acid-derived and contained in a target pharmaceutical product; converting the target sequence information into target vector data reflecting a three-dimensional structure of the amino acids; deriving target formulation information representing a formulation of a storage solution suitable for the target pharmaceutical product based on the target vector data; and presenting the target formulation information to a user.

An operation program of a pharmaceutical product development support device according to the present disclosure causes a computer to execute a process comprising: acquiring target sequence information representing a sequence of amino acids constituting a substance that is amino acid-derived and contained in a target pharmaceutical product; converting the target sequence information into target vector data reflecting a three-dimensional structure of the amino acids; deriving target formulation information representing a formulation of a storage solution suitable for the target pharmaceutical product based on the target vector data; and presenting the target formulation information to a user.

According to the technology of the present disclosure, it is possible to provide a pharmaceutical product development support device, an operation method of a pharmaceutical product development support device, and an operation program of a pharmaceutical product development support device, which can improve prediction accuracy of a formulation of a storage solution.

### BRIEF DESCRIPTION OF THE DRAWINGS

FIG. 1 is a diagram showing a pharmaceutical product development support system.
FIG. 2 is a diagram showing a composition of a biopharmaceutical product.
FIG. 3 is a diagram showing target sequence information.
FIG. 4 is a diagram showing target formulation information.
FIG. 5 is a block diagram showing a pharmaceutical product development support device and a computer constituting a user terminal.
FIG. 6 is a block diagram showing a processing unit of a CPU of the pharmaceutical product development support device.
FIG. 7 is a diagram showing a composition of a conversion model.
FIG. 8 is a diagram showing processing of a conversion unit.
FIG. 9 is a diagram showing a composition of training data of a prediction model.
FIG. 10 is a diagram showing reference formulation information.
FIG. 11 is a diagram showing a state in which the reference formulation information is clustered and cluster information.
FIG. 12 is a diagram showing a state in which appearance frequency information is generated from the cluster information.
FIG. 13 is a diagram illustrating processing in a learning phase of a prediction model.
FIG. 14 is a diagram showing processing of a derivation unit.
FIG. 15 is a diagram showing processing of a derivation unit.
FIG. 16 is a block diagram showing a processing unit of a CPU of the user terminal.
FIG. 17 is a diagram showing a target sequence information input screen.
FIG. 18 is a diagram showing a prediction result display screen.
FIG. 19 is a flowchart showing a processing procedure of the pharmaceutical product development support device.
FIG. 20 is a diagram showing Embodiment 2_1 in which the number of pieces of the target formulation information to be derived is designated as the derivation condition.
FIG. 21 is a diagram showing processing of the derivation unit in Embodiment 2_1.
FIG. 22 is a diagram showing Embodiment 2_2 in which the type of the buffer solution is designated as the derivation condition.
FIG. 23 is a diagram showing processing of the derivation unit in Embodiment 2_2.

### DETAILED DESCRIPTION

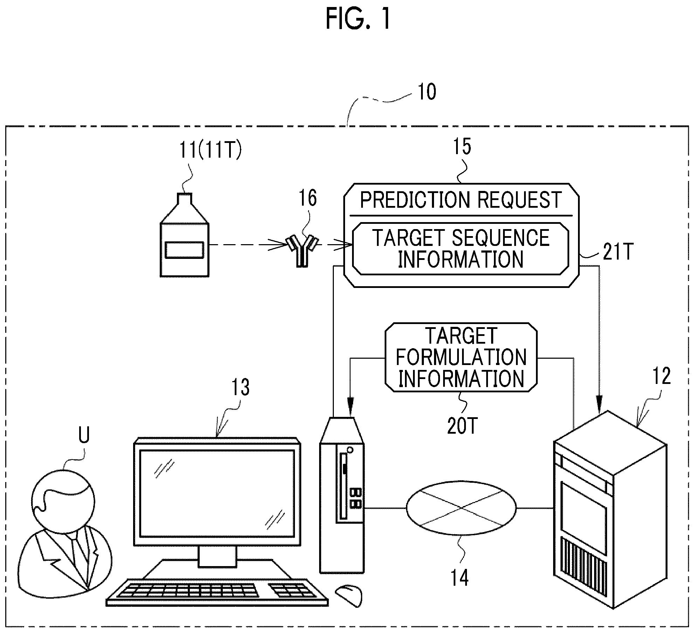

As shown in FIG. 1 as an example, a pharmaceutical product development support system 10 is a system that supports the development of a biopharmaceutical product 11, and comprises a pharmaceutical product development support device 12 and a user terminal 13. The pharmaceutical product development support device 12 and the user terminal 13 are connected to each other via a network 14. The user terminal 13 is installed in a pharmaceutical company that develops the biopharmaceutical product 11 or in an institution that receives a pharmaceutical development service of the biopharmaceutical product 11 from the pharmaceutical company, that is, a contract research organization (CRO). The user terminal 13 is operated by a user U who is involved in the development of the biopharmaceutical product 11 in the pharmaceutical company or the CRO (hereinafter, collectively referred to as a pharmaceutical facility). The network 14 is, for example, a wide area network (WAN) such as the Internet or a public communication network. In FIG. 1, only one user terminal 13 is connected to the pharmaceutical product development support device 12, but in practice, a plurality of user terminals 13 of a plurality of pharmaceutical facilities are connected to the pharmaceutical product development support device 12.

The user terminal 13 transmits a prediction request 15 to the pharmaceutical product development support device 12. The prediction request 15 is a request for the pharmaceutical product development support device 12 to predict information useful for promoting the development of the biopharmaceutical product 11.

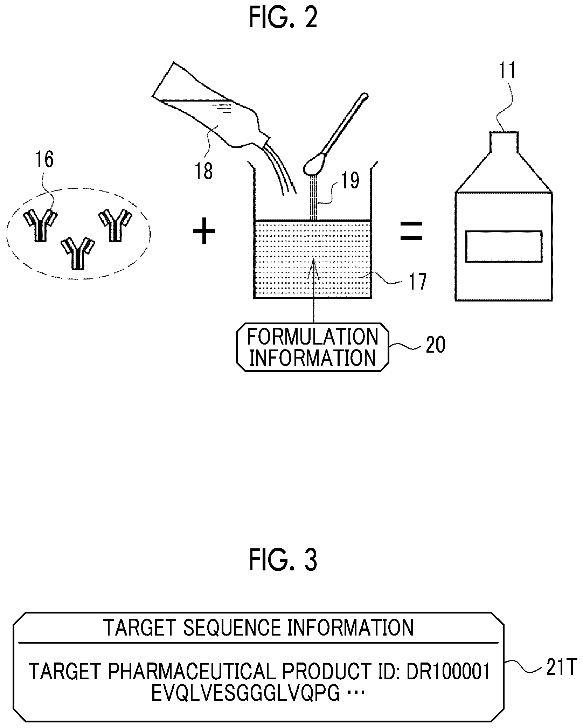

As shown in FIG. 2 as an example, the biopharmaceutical product 11 is a mixed solution of an antibody 16, which is an active ingredient, and a storage solution 17. The antibody 16 is an example of an "amino acid-derived substance" according to the technology of the present disclosure.

The storage solution 17 is composed of a base buffer solution 18 and an additive 19 added to the buffer solution 18. The additive 19 is a salt, a sugar, an amino acid, and a surfactant. The storage solution 17 is prepared with reference to formulation information 20 that defines the type of the buffer solution 18 and the presence or absence of the salt, the sugar, the amino acid, and the surfactant.

There are a plurality of types of buffer solutions 18, such as a phosphate buffer solution, an acetate buffer solution, and a citrate buffer solution. The salt is, for example, sodium chloride, potassium chloride, sodium acetate, ammonium sulfate, or the like. The sugar is, for example, a pure sugar such as purified white sugar, sucrose, and trehalose, a sugar alcohol such as glycerin and sorbitol, or the like. The amino acid is proline, arginine, glutamine, histidine, or the like. The surfactant is polysorbate 20, polysorbate 80, or the like.

Returning to FIG. 1, the prediction request 15 includes target sequence information 21T. The target sequence information 21T is information representing a sequence of amino acids constituting the antibody 16 included in the target pharmaceutical product 11T. The target sequence information 21T is identified by an experiment. Here, the target pharmaceutical product 11T is the biopharmaceutical product 11 currently being developed by the user U in the pharmaceutical facility, and is the biopharmaceutical product 11 of which the formulation of the suitable storage solution 17 is unknown. Although not shown, the prediction request 15 also includes a terminal ID (identification data) or the like for uniquely identifying the user terminal 13 that is a transmission source of the prediction request 15.

In a case where the prediction request 15 is received, the pharmaceutical product development support device 12 predicts the target formulation information 20T representing the formulation of the storage solution 17 suitable for the target pharmaceutical product 11T as information useful for promoting the development of the biopharmaceutical product 11. Then, the target formulation information 20T is delivered to the user terminal 13 as the transmission source of the prediction request 15. In a case where the target formulation information 20T is received, the user terminal 13 provides the target formulation information 20T for viewing by the user U.

As shown in FIG. 3 as an example, the target sequence information 21T includes a target pharmaceutical product ID for uniquely identifying the target pharmaceutical product 11T. Then, the target sequence information 21T is obtained by describing the order of peptide bonds of the amino acids constituting the antibody 16 included in the target pharmaceutical product 11T from an amino terminal to a carboxyl terminal using abbreviations of one-letter alphabets representing the amino acids. Since the number of amino acids constituting the antibody 16 is approximately 450, the number of alphabets in the target sequence information 21T is also approximately 450. For example, the abbreviation "E" is glutamic acid, the abbreviation "L" is leucine, and the abbreviation "G" is glycine. Such a sequence of amino acids is also referred to as a primary structure.

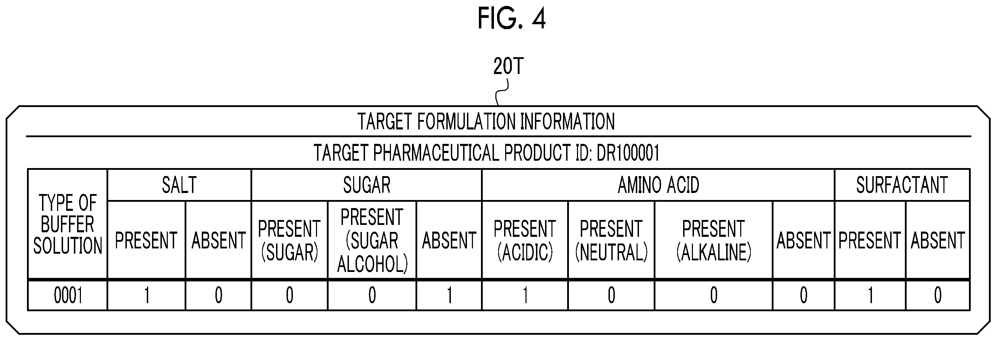

As shown in FIG. 4 as an example, the target formulation information 20T includes the target pharmaceutical product ID, similarly to the target sequence information 21T. Then, the target formulation information 20T is information in which the type of the buffer solution 18 and the presence or absence of the salt, the sugar, the amino acid, and the surfactant are represented by multidimensional (here, 15-dimensional) vector data consisting of a sequence of binary numbers of "0" and "1". The type of the buffer solution 18 is represented by a 4-bit binary number such as "0001". The salt has items of "present" and "absent". In a case where the addition of the salt is appropriate, "1" is registered in "present" and "0" is registered in "absent". On the other hand, in a case where the addition of the salt is not appropriate, "0" is registered in "present" and "1" is registered in "absent". The sugar has items of "present (sugar)", "present (sugar alcohol)", and "absent". In a case where the addition of the sugar is appropriate, "1" is registered in "present (sugar)" and "0" is registered in the other items. In addition, in a case where the addition of the sugar alcohol is appropriate, "1" is registered in "present (sugar alcohol)" and "0" is registered in the other items. On the other hand, in a case where the addition of the sugar and the sugar alcohol is not appropriate, "1" is registered in "absent" and "0" is registered in the other items.

The amino acid has items of "present (acidic)", "present (neutral)", "present (alkaline)", and "absent". In a case where the addition of the acidic amino acid is appropriate, "1" is registered in "present (acidic)" and "0" is registered in the other items. In a case where the addition of the neutral amino acid is appropriate, "1" is registered in "present (neutral)" and "0" is registered in the other items. In addition, in a case where the addition of the alkaline amino acid is appropriate, "1" is registered in "present (alkaline)" and "0" is registered in the other items. On the other hand, in a case where the addition of the amino acid is not appropriate, "1" is registered in "absent" and "0" is registered in the other items. The surfactant has items of "present" and "absent". In a case where the addition of the surfactant is appropriate, "1" is registered in "present" and "0" is registered in "absent". On the other hand, in a case where the addition of the surfactant is not appropriate, "0" is registered in "present" and "1" is registered in "absent".

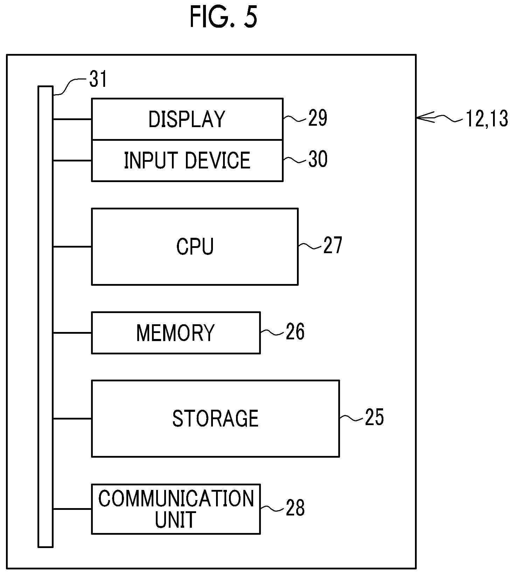

As shown in FIG. 5 as an example, the computers constituting the pharmaceutical product development support device 12 and the user terminal 13 basically have the same configuration, and comprise a storage 25, a memory 26, a central processing unit (CPU) 27, a communication unit 28, a display 29, and an input device 30. These units are connected to each other through a busline 31.

The storage 25 is a hard disk drive that is built in the computers constituting the pharmaceutical product development support device 12 and the user terminal 13 or is connected to the computers via a cable or a network. Alternatively, the storage 25 is a disk array in which a plurality of hard disk drives are connected in series. The storage 25 stores a control program such as an operating system, various application programs (hereinafter, referred to as an application program (AP)), various types of data associated with these programs, and the like. Note that a solid-state drive may be used instead of the hard disk drive.

The memory 26 is a work memory which is necessary to execute processing by the CPU 27. The CPU 27 loads the program stored in the storage 25 into the memory 26, and executes processing according to the program. Accordingly, the CPU 27 controls each unit of the computer in an integrated manner. The CPU 27 is an example of a "processor" according to the technology of the present disclosure. Note that the memory 26 may be incorporated into the CPU 27.

The communication unit 28 is a network interface that controls transmission of various types of information through the network 14 or the like. The display 29 displays various screens. The various screens have an operation function using a graphical user interface

(GUI). The computers constituting the pharmaceutical product development support device 12 and the user terminal 13 receive an input of an operation instruction from an input device 30 through various screens. The input device 30 is a keyboard, a mouse, a touch panel, a microphone for audio input, or the like.

In the following description, each unit (the storage 25 and the CPU 27) of the computer constituting the pharmaceutical product development support device 12 is distinguished by adding a subscript "A" to the reference numeral, and each unit (the storage 25, the CPU 27, the display 29, and the input device 30) of the computer constituting the user terminal 13 is distinguished by adding a subscript "B" to the reference numeral.

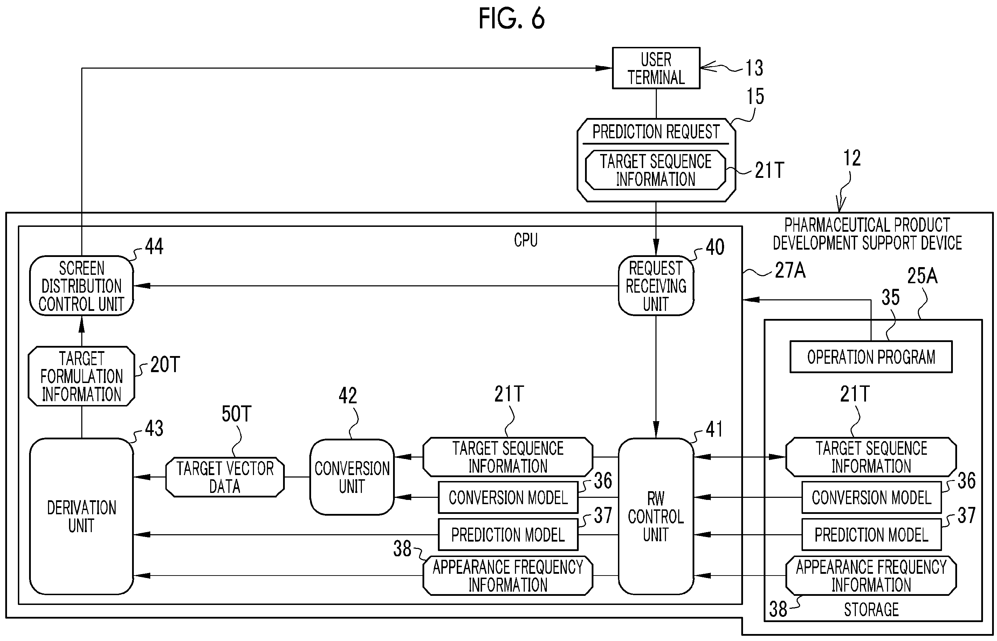

As shown in FIG. 6 as an example, an operation program 35 is stored in a storage 25A of the pharmaceutical product development support device 12. The operation program 35 is an AP for causing the computer to function as the pharmaceutical product development support device 12. That is, the operation program 35 is an example of an "operation program for a pharmaceutical product development support device" according to the technology of the present disclosure. A conversion model 36, a prediction model 37, appearance frequency information 38, and the like are also stored in the storage 25A. The prediction model 37 is an example of a "machine learning model" according to the technology of the present disclosure.

In a case where the operation program 35 is started, a CPU 27A of the computer constituting the pharmaceutical product development support device 12 functions as a request receiving unit 40, a read/write (hereinafter, abbreviated as RW) control unit 41, a conversion unit 42, a derivation unit 43, and a screen distribution control unit 44 in cooperation with the memory 26 and the like.

The request receiving unit 40 receives various requests from the user terminal 13. In particular, the request receiving unit 40 receives a prediction request 15 from the user terminal 13. The prediction request 15 includes the target sequence information 21T as described above. Therefore, the request receiving unit 40 receives the prediction request 15 to acquire the target sequence information 21T. In a case where the prediction request 15 is received, the request receiving unit 40 outputs the target sequence information 21T included in the prediction request 15 to the RW control unit 41. In addition, the request receiving unit 40 outputs a terminal ID of the user terminal 13 included in the prediction request 15 to the screen distribution control unit 44.

The RW control unit 41 controls storage of various types of data in the storage 25A and readout of various types of data from the storage 25A. For example, the RW control unit 41 stores the target sequence information 21T from the request receiving unit 40 in the storage 25A. In addition, the RW control unit 41 reads out the target sequence information 21T from the storage 25A and outputs the readout target sequence information 21T to the conversion unit 42.

Further, the RW control unit 41 reads out the conversion model 36 from the storage 25A and outputs the readout conversion model 36 to the conversion unit 42. In addition, the RW control unit 41 reads out the prediction model 37 and the appearance frequency information 38 from the storage 25A and outputs the readout prediction model 37 and the readout appearance frequency information 38 to the derivation unit 43.

The conversion unit 42 converts the target sequence information 21T into target vector data 50T by using the conversion model 36. The conversion unit 42 outputs the target vector data 50T to the derivation unit 43.

The derivation unit 43 derives the target formulation information 20T corresponding to the target vector data 50T by using the prediction model 37 and the appearance frequency information 38. The derivation unit 43 outputs the target formulation information 20T to the screen distribution control unit 44.

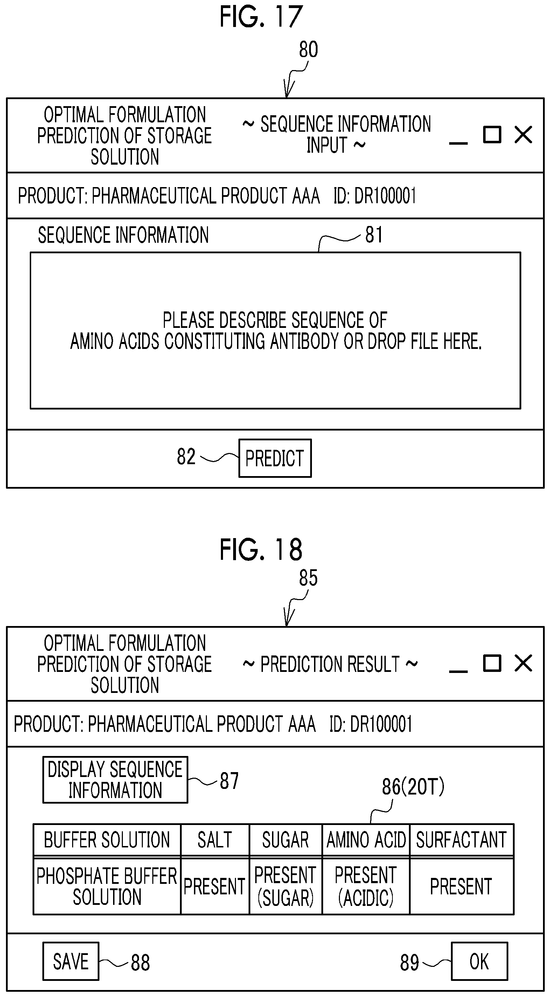

The screen distribution control unit 44 performs control of distributing various screens to the user terminal 13. Specifically, the screen distribution control unit 44 distributes and outputs various screens to the user terminal 13, which is a transmission source of various requests, in a format of screen data for web distribution created by a markup language such as extensible markup language (XML). In this case, the screen distribution control unit 44 specifies the user terminal 13, which is the transmission source of various requests, based on the terminal ID from the request receiving unit 40. The various screens include a target sequence information input screen 80 (see FIG. 17) for inputting the target sequence information 21T, a prediction result display screen 85 (see FIG. 18) for displaying the target formulation information 20T, and the like. Another data description language such as JavaScript (registered trademark) Object Notation (JSON) may be used instead of XML.

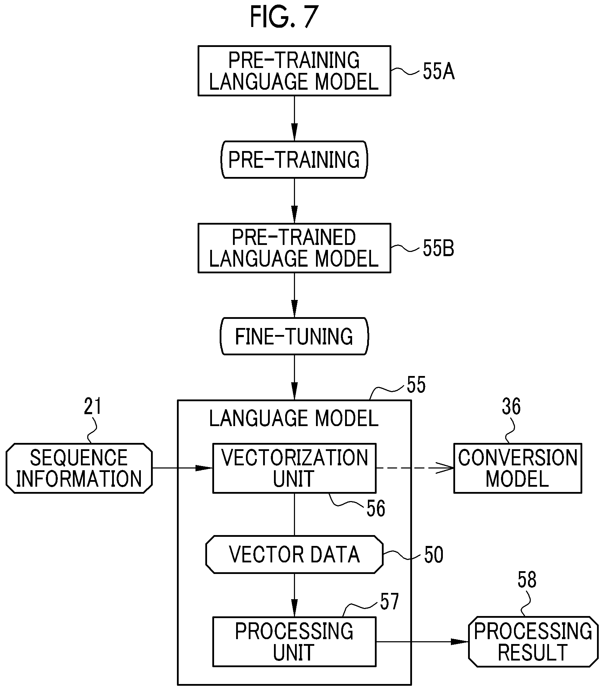

As shown in FIG. 7 as an example, a vectorization unit 56, which is a part of the language model 55, is diverted to the conversion model 36. The language model 55 is a machine learning model that has been originally used in the field of natural language processing (NLP). The language model 55 treats the sequence information 21 of the amino acids constituting the antibody 16 as a sentence. The language model 55 is based on, for example, bidirectional encoder representations from transformers (BERT) using a transformer encoder.

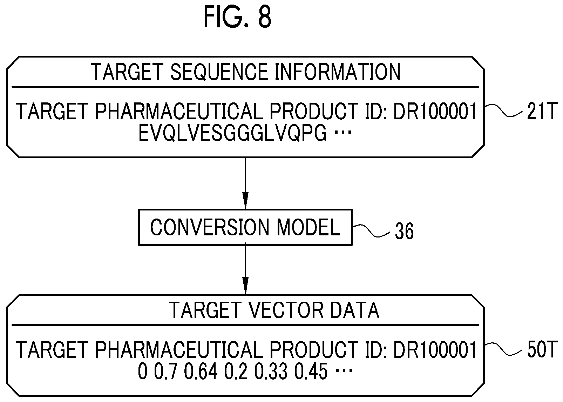

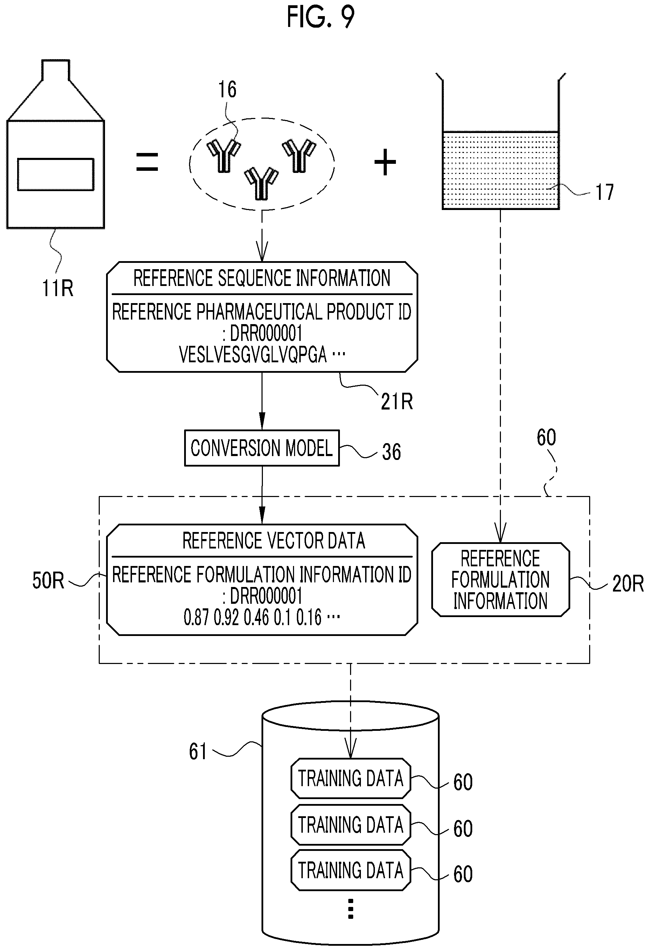

The vectorization unit 56 converts the sequence information 21 input to the language model 55 into vector data 50. The vector data 50 is, for example, multidimensional data consisting of a sequence of real number values between 0 and 1 (see FIGS. 8 and 9). The number of dimensions of the vector data 50 is, for example, 512, 1024, 2048, or the like. The vector data 50 reflects not only the sequence (primary structure) of the amino acids constituting the antibody 16 but also the three-dimensional structure (secondary structure, tertiary structure, and quaternary structure). The vectorization unit 56 outputs the vector data 50 to a processing unit 57. The processing unit 57 outputs a processing result 58 based on the vector data 50.

The language model 55 is generated by performing pre-training on a pre-training language model 55A and further performing fine-tuning on a pre-trained language model 55B. The pre-training includes masked language modeling (MLM) and next sentence prediction (NSP). The MLM is a learning in which a part of the sequence of the amino acids of the sequence information 21 is masked and the amino acid to be entered in the masked portion is predicted, and is a so-called cloze task. The NSP is a learning in which it is determined whether or not the sequence information 21 of the amino acids constituting two different antibodies 16 is relevant. The fine-tuning is a learning according to a desired processing task to be performed by the processing unit 57. The processing task is, for example, a task of outputting an appropriate value of the hydrogen ion exponent (potential of hydrogen (pH) value) of the storage solution 17 or an appropriate value of the temperature of the storage solution 17 as the processing result 58 in response to the input of the sequence information 21. The vectorization unit 56 of the language model 55 generated in this way is stored in the storage 25A as the conversion model 36. The pre-training and the fine-tuning may be performed in the pharmaceutical product development support device 12 or may be performed by a device different from the pharmaceutical product development support device 12. In addition, the pre-training and the fine-tuning may be continued even after the conversion model 36 is stored in the storage 25A. In addition, the fine-tuning need not be performed by performing only the pre-training. Further, the NSP may not be performed.

As shown in FIG. 8 as an example, the conversion unit 42 inputs the target sequence information 21T to the conversion model 36 and outputs the target vector data 50T from the conversion model 36. The target vector data 50T includes the target pharmaceutical product ID, similarly to the target sequence information 21T and the like.

Hereinafter, the training of the prediction model 37 will be described. First, as shown in FIG. 9 as an example, the training data 60 of the prediction model 37 is generated from the reference pharmaceutical product 11R. The reference pharmaceutical product 11R is the biopharmaceutical product 11 that is different from the target pharmaceutical product 11T and that is developed earlier than the target pharmaceutical product 11T, and is the biopharmaceutical product 11 of which the suitable formulation of the storage solution 17 is known. The reference sequence information 21R representing the sequence of the amino acids constituting the antibody 16 included in the reference pharmaceutical product 11R is converted into the reference vector data 50R by using the conversion model 36. Then, a set of the reference vector data 50R and the reference formulation information 20R representing the formulation of the storage solution 17 suitable for the reference pharmaceutical product 11R is stored in the database 61 as the training data 60. The database 61 stores a plurality of pieces of training data 60 generated from a plurality of reference pharmaceutical products 11R.

The reference sequence information 21R and the reference vector data 50R include the reference pharmaceutical product ID for uniquely identifying the reference pharmaceutical product 11R. The reference sequence information 21R is described from the amino terminal to the carboxyl terminal using the one-letter abbreviation of the alphabet representing the amino acid, in the same manner as the target sequence information 21T, in which the order of the peptide bonds of the amino acids constituting the antibody 16 included in the reference pharmaceutical product 11R is described. In addition, the reference vector data 50R consists of, for example, a sequence of real number values between 0 and 1 that reflect the three-dimensional structure of the amino acids constituting the antibody 16 included in the reference pharmaceutical product 11R, in the same manner as the target vector data 50T.

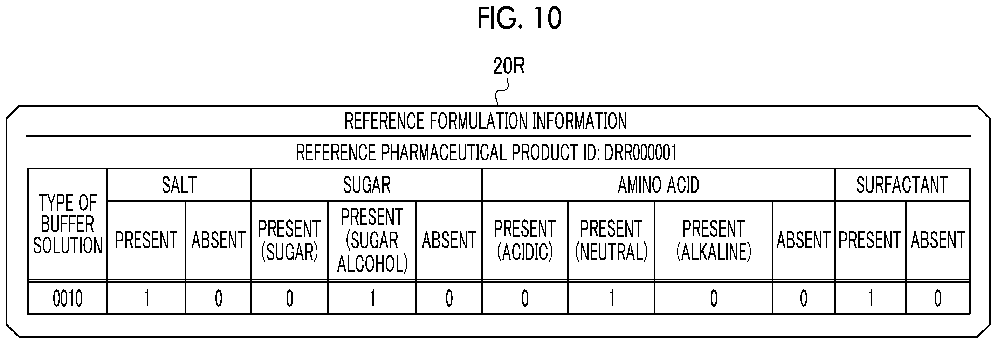

As shown in FIG. 10 as an example, the reference formulation information 20R includes the reference pharmaceutical product ID, similarly to the reference sequence information 21R and the like. The reference formulation information 20R is information in which the type of the buffer solution 18 and the presence or absence of the salt, the sugar, the amino acid, and the surfactant are represented by vector data consisting of a sequence of binary numbers of "0" and "1", similarly to the target formulation information 20T.

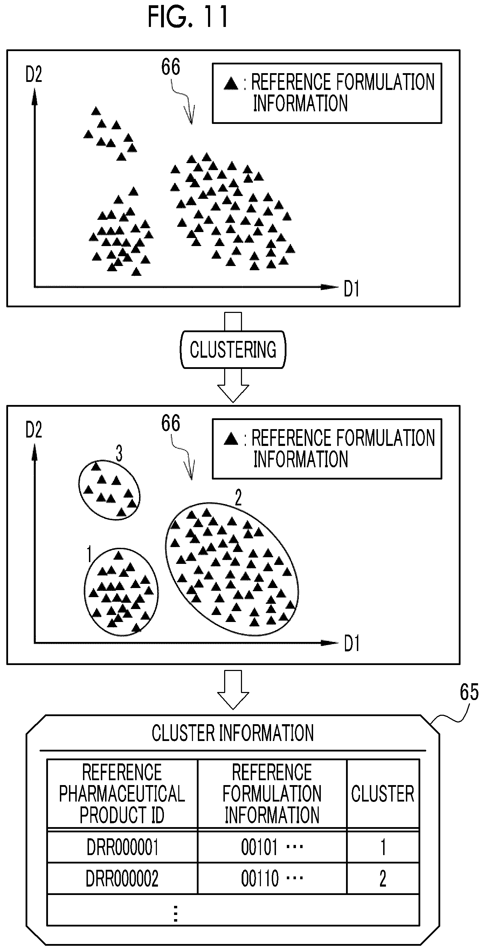

After the collection of the training data 60, as shown in FIG. 11 as an example, clustering processing of defining a cluster to which each of a plurality of pieces of reference formulation information 20R belongs is performed. FIG. 11 shows an example in which the plurality of pieces of reference formulation information 20R are clustered into three clusters of a cluster 1, a cluster 2, and a cluster 3. As the clustering processing, a k-means method or hierarchical clustering can be used. As a result of the clustering processing, cluster information 65 is generated. The cluster information 65 is information in which the cluster to which each reference pharmaceutical product ID and each reference formulation information 20R belongs is registered. In FIG. 11, for convenience of description, the dimension of the vector space 66 in which the reference formulation information 20R is plotted is set to two dimensions having a D1 axis and a D2 axis, but the actual dimension of the vector space 66 is, for example, 512 dimensions as described above. Instead of the reference formulation information 20R, the clustering processing may be performed on the reference vector data 50R. In addition, the clustering processing may be performed on the vector data in which the reference formulation information 20R and the reference vector data 50R are combined.

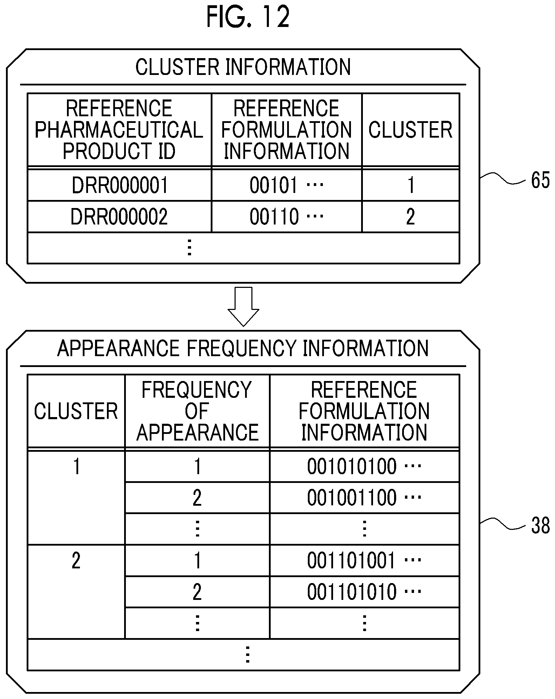

After the clustering processing, as shown in FIG. 12 as an example, appearance frequency information 38 is generated based on the cluster information 65. More specifically, the number of times of appearance of the reference formulation information 20R of each cluster is counted. Then, the reference formulation information 20R is arranged in descending order of the number of times of appearance for each cluster to obtain the appearance frequency information 38. The frequencies of appearance of "1", "2", ... in the appearance frequency information 38 represent the ranks of the number of times of appearance. For example, the reference formulation information 20R having the highest number of times of appearance and the highest frequency of appearance in the cluster 1 is "001010100...". In addition, the reference formulation information 20R having the second highest number of times of appearance and the second frequency of appearance in the cluster 2 is "001101010...". The appearance frequency information 38 generated in this way is stored in the storage 25A.

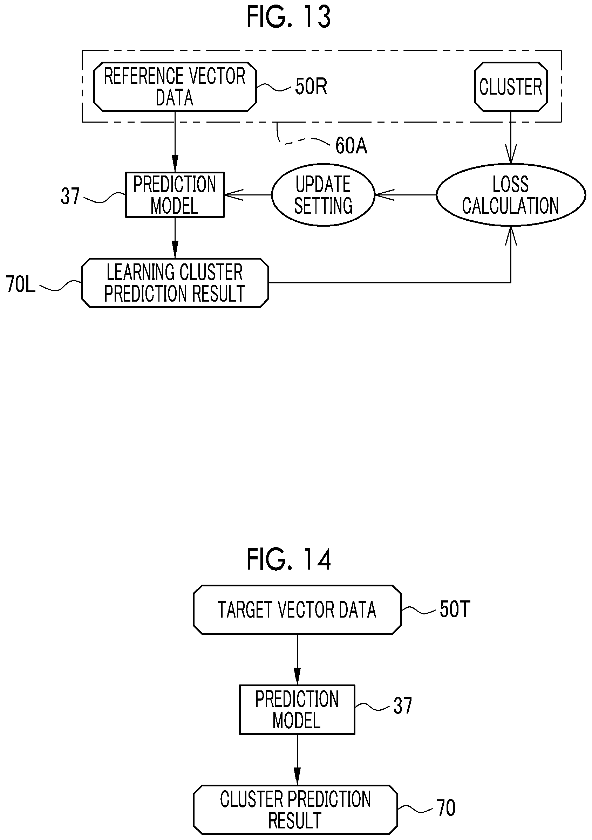

As shown in FIG. 13 as an example, in the learning of the prediction model 37, training data 60A composed of a set of the reference vector data 50R and the cluster derived from the training data 60 is used. Specifically, the reference vector data 50R is input to the prediction model 37, and thus a learning cluster prediction result 70L is output from the prediction model 37. The learning cluster prediction result 70L is a prediction result of the cluster to which the reference formulation information 20R corresponding to the original reference pharmaceutical product 11R of the reference vector data 50R belongs. A loss calculation of the prediction model 37 using a loss function is performed based on the learning cluster prediction result 70L and the cluster of the training data 60A. Then, update settings of various coefficients (coefficients of the filter of the convolutional layer, and the like) of the prediction model 37 are performed according to a result of the loss calculation, and the prediction model 37 is updated according to the update settings.

In the learning of the prediction model 37, the series of processing of the input of the reference vector data 50R to the prediction model 37, the output of the learning cluster prediction result 70L from the prediction model 37, the loss calculation, the update settings, and the update of the prediction model 37 is repeatedly performed while the training data 60A is replaced. The repetition of the series of processing is ended in a case where the prediction accuracy of the learning cluster prediction result 70L reaches a predetermined set level. The prediction model 37 of which the prediction accuracy reaches the set level is stored in the storage 25A. It should be noted that the learning may be ended in a case where the series of processing is repeated a set number of times regardless of the prediction accuracy of the learning cluster prediction result 70L. The learning of the prediction model 37 may be performed in the pharmaceutical product development support device 12 or may be performed by a device different from the pharmaceutical product development support device 12. Training of the prediction model 37 may continue even after storing the prediction model 37 in the storage 25A.

As shown in FIG. 14 as an example, the derivation unit 43 inputs the target vector data 50T to the prediction model 37 and outputs the cluster prediction result 70 from the prediction model 37. The cluster prediction result 70 is a prediction result of a cluster to which the target formulation information 20T corresponding to the original target pharmaceutical product 11T of the target vector data 50T belongs. It should be noted that the prediction model 37 is constructed by, for example, a machine learning algorithm such as a support-vector machine (SVM) or XGBoost.

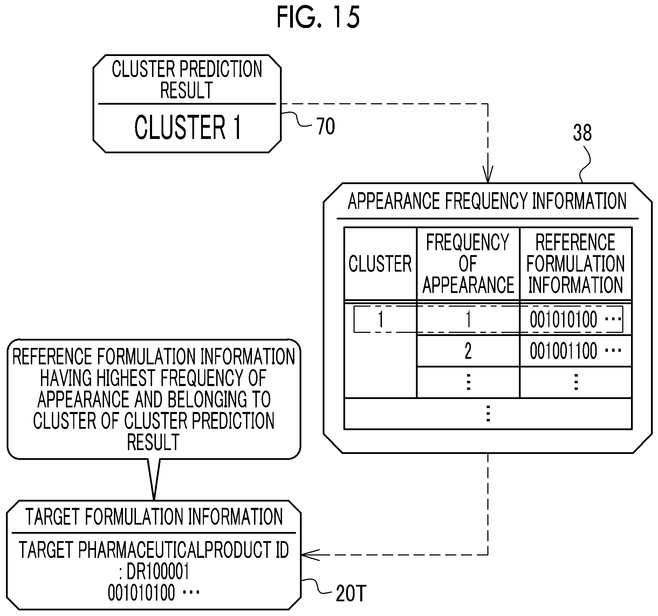

As shown in FIG. 15 as an example, the derivation unit 43 derives the reference formulation information 20R having the highest frequency of appearance among the reference formulation information 20R belonging to the cluster of the cluster prediction result 70 as the target formulation information 20T. FIG. 15 shows a case where the cluster of the cluster prediction result 70 is the cluster 1 and the reference formulation information 20R of "001010100..." having the highest frequency of appearance in the cluster 1 in the appearance frequency information 38 is derived as the target formulation information 20T. As described above, the derivation unit 43 derives the reference formulation information 20R belonging to the cluster output by the prediction model 37 as the target formulation information 20T. In addition, the derivation unit 43 derives the target formulation information 20T based on the frequency of appearance of the reference formulation information 20R belonging to the cluster output by the prediction model 37.

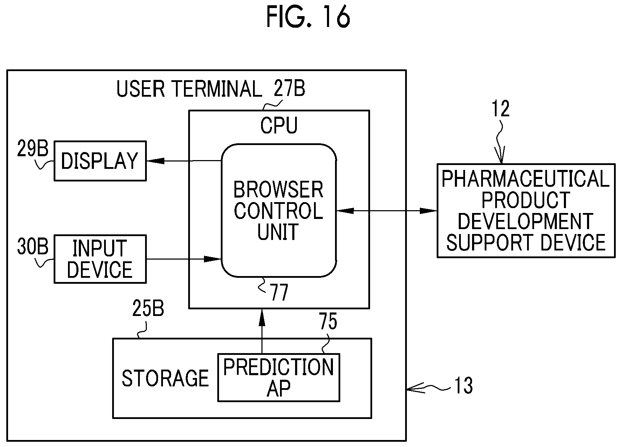

As shown in FIG. 16 as an example, a prediction AP 75 is stored in the storage 25B of the user terminal 13. The prediction AP 75 is installed in the user terminal 13 by the user U. The prediction AP 75 is an AP for predicting the information on the formulation of the storage solution 17 suitable for the target pharmaceutical product 11T, that is, the target formulation information 20T. In a case in which the prediction AP 75 is activated, a CPU 27B of the user terminal 13 functions as a browser control unit 77 in cooperation with the memory 26 and the like. The browser control unit 77 controls an operation of a dedicated web browser of the prediction AP 75.

The browser control unit 77 reproduces various screens based on various screen data from the pharmaceutical product development support device 12 and displays the reproduced various screens on the display 29B. In addition, the browser control unit 77 receives various operation instructions which are input from the input device 30B by the user U through various screens. The browser control unit 77 transmits various requests in response to the operation instructions, including the prediction request 15, to the pharmaceutical product development support device 12.

In a case where the prediction AP 75 is activated, a target sequence information input screen 80 shown in FIG. 17 as an example is displayed on the display 29B under the control of the browser control unit 77. The target sequence information input screen 80 is provided with an input box 81 for the target sequence information 21T. In the input box 81, the target sequence information 21T can be described or a file of the target sequence information 21T can be dropped.

The user U inputs desired target sequence information 21T into the input box 81 and then selects a prediction button 82. In a case where the prediction button 82 is selected, the browser control unit 77 generates the prediction request 15 including the target sequence information 21T input to the input box 81 and transmits the generated prediction request 15 to the pharmaceutical product development support device 12.

In addition, in a case where the prediction of the target formulation information 20T is performed in the pharmaceutical product development support device 12, a prediction result display screen 85 shown in FIG. 18 as an example is displayed on the display 29B under the control of the browser control unit 77. The prediction result display screen 85 displays the target formulation information 20T, more specifically, a table 86 showing the content of the target formulation information 20T. In this way, the target formulation information 20T is presented to the user U in the form of the distribution of the screen data.

A target sequence information display button 87 is provided at an upper part of the prediction result display screen 85. In a case where the target sequence information display button 87 is selected, a display screen of the target sequence information 21T is displayed in a pop-up manner. In addition, a save button 88 and an OK button 89 are provided at a lower part of the prediction result display screen 85. In a case where the save button 88 is selected, the target formulation information 20T is stored in the storage 25B in association with the target pharmaceutical product ID. In a case where the OK button 89 is selected, the display of the prediction result display screen 85 is turned off.

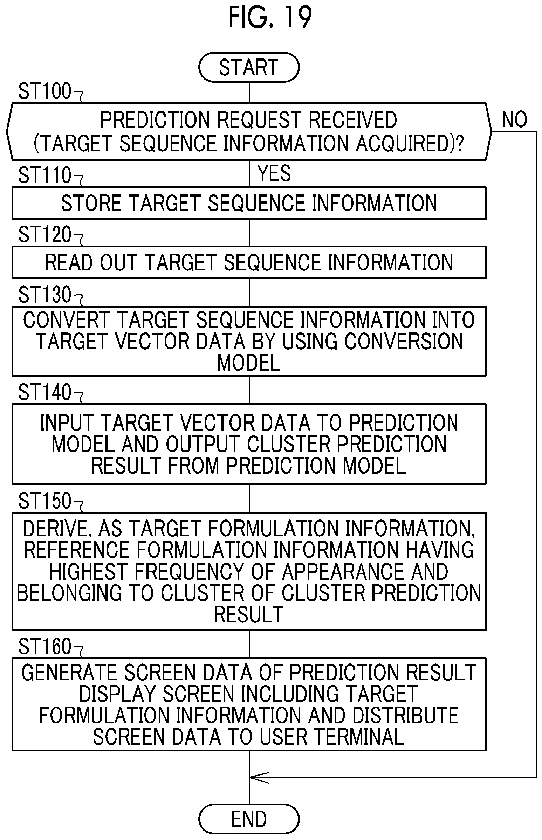

Next, an action with the configuration described above will be described with reference to the flowchart shown in FIG. 19 as an example. In a case where the operation program 35 is activated in the pharmaceutical product development support device 12, the CPU 27A functions as the request receiving unit 40, the RW control unit 41, the conversion unit 42, the derivation unit 43, and the screen distribution control unit 44 as shown in FIG. 6. In addition, in a case where the prediction AP 75 is activated in the user terminal 13, the CPU 27B functions as the browser control unit 77 as shown in FIG. 16.

The target sequence information input screen 80 shown in FIG. 17 is displayed on the display 29B of the user terminal 13 under the control of the browser control unit 77. In a case where the user U inputs the desired target sequence information 21T in the input box 81 on the target sequence information input screen 80 and selects the prediction button 82, the prediction request 15 is transmitted from the browser control unit 77 to the pharmaceutical product development support device 12. As shown in FIG. 1, the prediction request 15 includes the target sequence information 21T, the terminal ID of the user terminal 13, and the like.

In the pharmaceutical product development support device 12, the target sequence information 21T included in the prediction request 15 is acquired by receiving the prediction request 15 in the request receiving unit 40 (YES in step ST100). The target sequence information 21T included in the prediction request 15 is output from the request receiving unit 40 to the RW control unit 41 and is stored in the storage 25A under the control of the RW control unit 41 (step ST110). In addition, the terminal ID of the user terminal 13 included in the prediction request 15 is output from the request receiving unit 40 to the screen distribution control unit 44.

The target sequence information 21T is read out from the storage 25A by the RW control unit 41 (step ST120). The target sequence information 21T is output from the RW control unit 41 to the conversion unit 42. In addition, the conversion model 36 is read out from the storage 25A by the RW control unit 41, and the readout conversion model 36 is output to the conversion unit 42.

In the conversion unit 42, as shown in FIG. 8, the target sequence information 21T is input to the conversion model 36. As a result, the target vector data 50T is output from the conversion model 36. In this way, the target sequence information 21T is converted into the target vector data 50T by using the conversion model 36 (step ST130). The target vector data 50T is output from the conversion unit 42 to the derivation unit 43.

The prediction model 37 and the appearance frequency information 38 are read out from the storage 25A by the RW control unit 41, and the readout prediction model 37 and the readout appearance frequency information 38 are output to the derivation unit 43. Then, as shown in FIG. 14, the target vector data 50T is input to the prediction model 37 in the derivation unit 43. As a result, the cluster prediction result 70 is output from the prediction model 37 (step ST140). Subsequently, as shown in FIG. 15, the reference formulation information 20R having the highest frequency of appearance and belonging to the cluster of the cluster prediction result 70, is derived as the target formulation information 20T in the derivation unit 43 (step ST150). The target formulation information 20T is output from the derivation unit 43 to the screen distribution control unit 44.

The screen distribution control unit 44 generates the screen data of the prediction result display screen 85 shown in FIG. 18 based on the target formulation information 20T. The screen data of the prediction result display screen 85 is distributed to the user terminal 13, which is the transmission source of the prediction request 15, under the control of the screen distribution control unit 44 (step ST160).

In the user terminal 13, the screen data of the prediction result display screen 85 is reproduced under the control of the browser control unit 77, and the reproduced prediction result display screen 85 is displayed on the display 29B. As a result, the target formulation information 20T is presented to the user U.

As described above, the CPU 27A of the pharmaceutical product development support device 12 comprises the request receiving unit 40, the conversion unit 42, the derivation unit 43, and the screen distribution control unit 44. The request receiving unit 40 receives the prediction request 15 to acquire the target sequence information 21T. The conversion unit 42 converts the target sequence information 21T into the target vector data 50T reflecting the three-dimensional structure of the amino acid. The derivation unit 43 derives the target formulation information 20T representing the formulation of the storage solution 17 suitable for the target pharmaceutical product 11T based on the target vector data 50T. The screen distribution control unit 44 distributes the screen data of the prediction result display screen 85 including the target formulation information 20T to the user terminal 13 to present the target formulation information 20T to the user U. Since the target formulation information 20T is derived in consideration of the three-dimensional structure of the amino acid, it is possible to improve the prediction accuracy of the formulation of the storage solution 17.

As shown in FIG. 8, the conversion unit 42 converts the target sequence information 21T into the target vector data 50T by using the conversion model 36 which is a part of the language model 55 that treats the target sequence information 21T as a sentence in natural language processing. Therefore, it is possible to easily obtain the target vector data 50T that well reflects the three-dimensional structure of the amino acid.

As shown in FIG. 4, the target formulation information 20T is vector data representing the type of the buffer solution 18 that is a base of the storage solution 17 suitable for the target pharmaceutical product 11T and the presence or absence of the salt, the sugar, the amino acid, and the surfactant contained in the storage solution 17 suitable for the target pharmaceutical product 11T. According to the past knowledge, in a case where the type of the buffer solution 18 and the presence or absence of the salt, the sugar, the amino acid, and the surfactant are known, the preparation of the storage solution 17 suitable for the target pharmaceutical product 11T is easy. Therefore, the target formulation information 20T can be set to the minimum necessary information in which the information that is not so useful for the preparation of the storage solution 17 is discarded.

As shown in FIGS. 9, 13, 14, and 15, the derivation unit 43 derives the target formulation information 20T by using the prediction model 37 that has been trained by the training data 60A derived from the training data 60 composed of a set of the reference vector data 50R and the reference formulation information 20R. The reference vector data 50R is data converted from the reference sequence information 21R representing the sequence of the amino acids constituting the antibody 16 contained in the reference pharmaceutical product 11R different from the target pharmaceutical product 11T. In addition, the reference formulation information 20R is information representing the formulation of the storage solution 17 suitable for the reference pharmaceutical product 11R. Therefore, it is possible to easily derive the target formulation information 20T having high prediction accuracy.

As shown in FIG. 11, the reference formulation information 20R is clustered into one of a plurality of clusters. As shown in FIG. 14, the prediction model 37 outputs one cluster of the plurality of clusters as the cluster prediction result 70 in response to the input of the target vector data 50T. As shown in FIG. 15, the derivation unit 43 derives the reference formulation information 20R belonging to the cluster output by the prediction model 37 as the target formulation information 20T.

The number of combinations of the types of the buffer solutions 18, and the presence or absence of the salt, the sugar, the amino acid, and the surfactant is extremely large. It is difficult to predict one combination from the extremely large number of combinations, and even in a case where the prediction can be performed, there is a concern that the prediction accuracy may be low. On the other hand, the number of clusters is at least less than the number of combinations of the types of the buffer solutions 18, and the presence or absence of the salt, the sugar, the amino acid, and the surfactant, and there is also a possibility that the number of clusters can be narrowed down to several as in the present example. Therefore, it is possible to improve the prediction accuracy of the target formulation information 20T by predicting the cluster rather than predicting the combination of the types of the buffer solutions 18, and the presence or absence of the salt, the sugar, the amino acid, and the surfactant. In a case where there is no concern that the prediction accuracy may be low, the prediction model 37 that derives the target formulation information 20T itself in response to the input of the target vector data 50T instead of the cluster prediction result 70 may be used.

As shown in FIG. 15, the derivation unit 43 derives the target formulation information 20T based on the frequency of appearance of the reference formulation information 20R belonging to the cluster output by the prediction model 37. Therefore, it is possible to derive the target formulation information 20T having high validity.

The reference formulation information 20R is vector data representing the type of the buffer solution 18 that serves as a base of the storage solution 17 suitable for the reference pharmaceutical product 11R, and the presence or absence of the salt, the sugar, the amino acid, and the surfactant contained in the storage solution 17 suitable for the reference pharmaceutical product 11R. Therefore, as in the case of the target formulation information 20T, the reference formulation information 20R can be set to the minimum necessary information in which the information that is not so useful for preparing the storage solution 17 is discarded.

The biopharmaceutical product 11 containing the antibody 16 as the protein, which is called an antibody pharmaceutical product, is widely used for the treatment of rare diseases such as hemophilia and Crohn's disease in addition to the treatment of chronic diseases such as cancer, diabetes, and rheumatoid arthritis. Therefore, according to the present example in which the substance is a protein and the protein is the antibody 16, it is possible to further promote the development of antibody pharmaceutical products that are widely used for the treatment of various diseases.

In Embodiment 1 described above, an example has been described in which the reference formulation information 20R having the first rank of the frequency of appearance belonging to the cluster of the cluster prediction result 70 is derived as the target formulation information 20T, but the present disclosure is not limited to this. For example, three pieces of reference formulation information 20R having the first to third ranks of the frequency of appearance belonging to the cluster of the cluster prediction result 70 may be derived as the target formulation information 20T. Alternatively, all the pieces of reference formulation information 20R belonging to the cluster of the cluster prediction result 70 may be derived as the target formulation information 20T. In addition, the target formulation information 20T may be derived based on the derivation condition designated by the user U, as in Embodiment 2_1 and Embodiment 2_2 described below.

### [Embodiment 2_1]

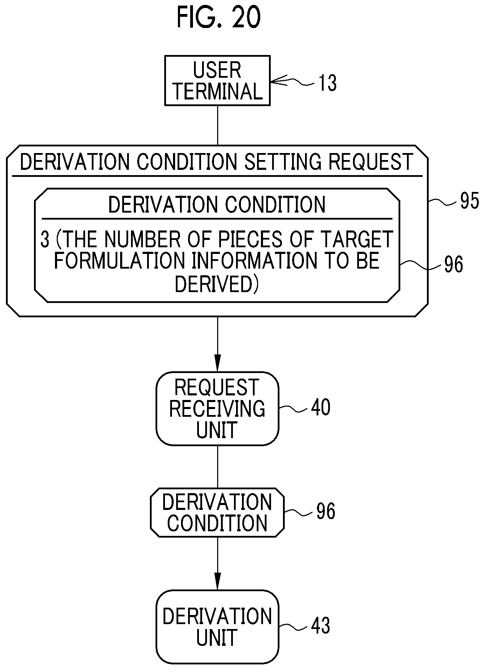

As shown in FIG. 20 as an example, in Embodiment 2_1, the request receiving unit 40 receives a derivation condition setting request 95 from the user terminal 13. The derivation condition setting request 95 includes a derivation condition 96 for designating the number of pieces of the target formulation information 20T to be derived. The derivation condition 96 is input by the user U through the input device 30B. The request receiving unit 40 outputs the derivation condition 96 included in the derivation condition setting request 95 to the derivation unit 43. FIG. 20 shows a case where "3" is designated as the derivation condition 96.

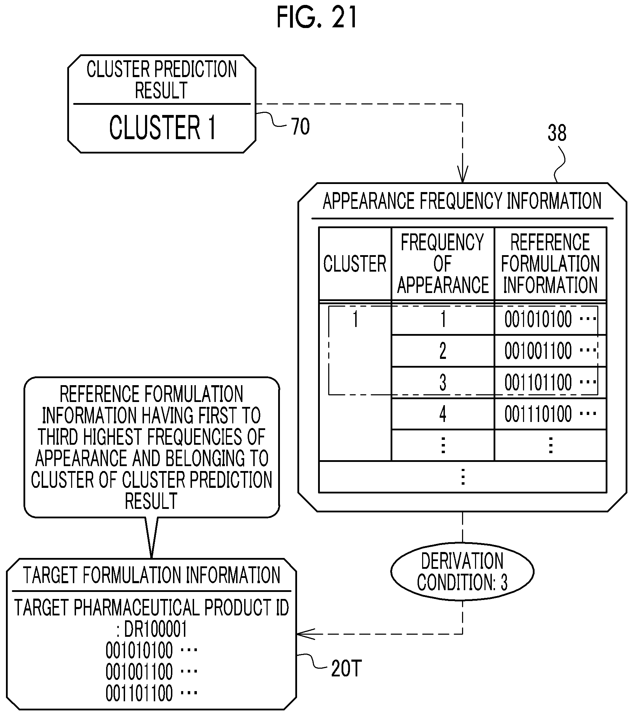

In this case, as shown in FIG. 21 as an example, the derivation unit 43 derives three pieces of reference formulation information 20R having the first to third highest frequencies of appearance and belonging to the cluster of the cluster prediction result 70 as the target formulation information 20T.

### [Embodiment 2_2]

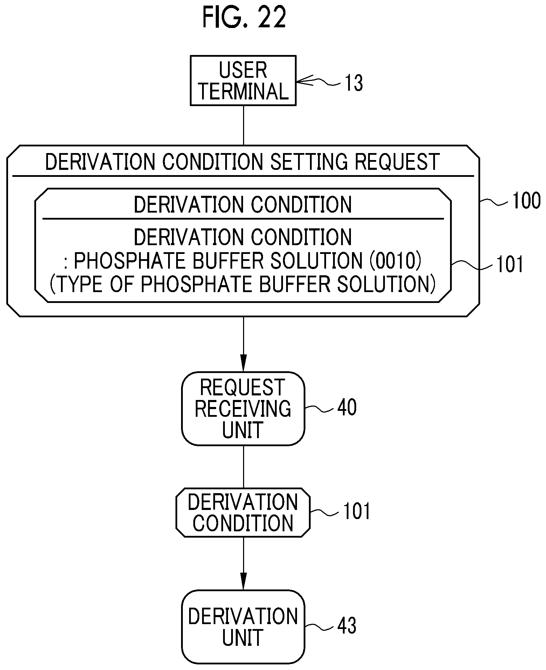

As shown in FIG. 22 as an example, in Embodiment 2_2, the request receiving unit 40 receives a derivation condition setting request 100 from the user terminal 13. The derivation condition setting request 100 includes a derivation condition 101 for designating the type of the buffer solution 18 and the presence or absence of the salt, the sugar, the amino acid, and the surfactant. The derivation condition 101 is input by the user U through the input device 30B. The request receiving unit 40 outputs the derivation condition 101 included in the derivation condition setting request 100 to the derivation unit 43. FIG. 22 shows a case where the type of the buffer solution 18, that is, "phosphate buffer solution (0010)" is designated as the derivation condition 101.

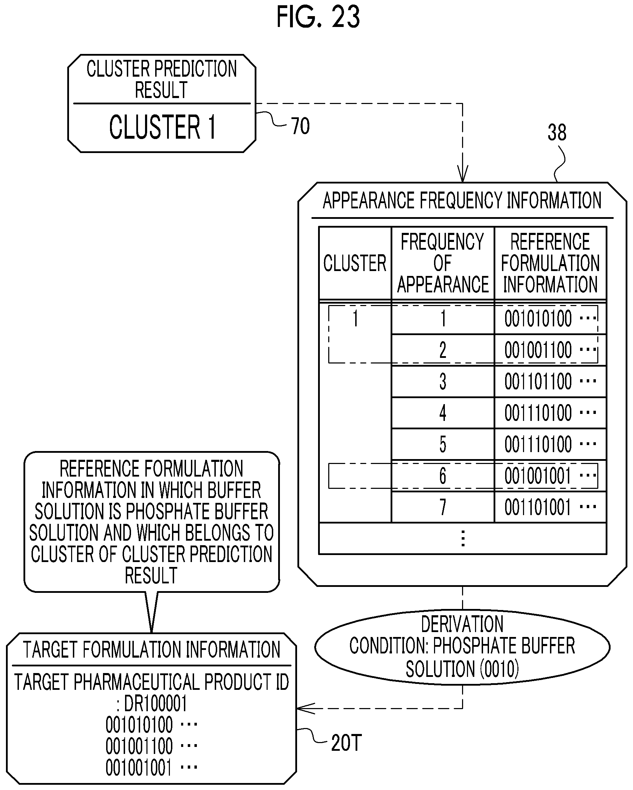

In this case, as shown in FIG. 23 as an example, the derivation unit 43 derives the reference formulation information 20R in which the buffer solution 18 is the phosphate buffer solution, which belongs to the cluster of the cluster prediction result 70, as the target formulation information 20T.

As described above, in Embodiment 2_1 and Embodiment 2_2, the request receiving unit 40 receives the designation of the derivation condition 96 or 101 of the target formulation information 20T by the user U. The derivation unit 43 derives the target formulation information 20T based on the derivation condition 96 or 101. Therefore, the target formulation information 20T that matches the intention of the user U can be derived.

In Embodiment 2_1, the derivation condition 96 is the number of the target formulation information 20T to be derived. Therefore, the number of the target formulation information 20T that matches the intention of the user U can be derived. In Embodiment 2_2, the derivation condition 101 is at least any one of the type of the buffer solution 18, or the presence or absence of the salt, the sugar, the amino acid, and the surfactant. Therefore, in a case where the user U has a certain level of knowledge about the formulation of the storage solution 17 suitable for the target pharmaceutical product 11T, such as the fact that the buffer solution 18 is considered to be only the phosphate buffer solution, the target formulation information 20T that matches the knowledge of the user U can be derived. In other words, the target formulation information 20T that does not match the knowledge of the user U can be excluded.

In Embodiment 2_1, three pieces of the reference formulation information 20R randomly selected from the cluster of the cluster prediction result 70 may be derived as the target formulation information 20T regardless of the frequency of appearance. In addition, in Embodiment 2_2, a composite derivation condition 101 may be designated, such as designating the type of the buffer solution and the presence or absence of the salt in the derivation condition 101. Further, Embodiment 2_1 and Embodiment 2_2 may be implemented in combination.

The protein is not limited to the antibody 16 given as an example. Examples of the protein include cytokine (interferon, interleukin, or the like), hormone (insulin, glucagon, follicle-stimulating hormone, erythropoietin, or the like), a growth factor (insulin-like growth factor (IGF)-1, basic fibroblast growth factor (bFGF), or the like), a blood coagulation factor (a seventh factor, an eighth factor, a ninth factor, or the like), an enzyme (a lysosomal enzyme, a deoxyribonucleic acid (DNA) degrading enzyme, or the like), a fragment crystallizable (Fc) fusion protein, a receptor, albumin, and a protein vaccine. Examples of the antibody 16 include a bispecific antibody, an antibody drug conjugate, a low-molecular-weight antibody, and a sugar-chain-modified antibody.

In addition, the amino acid-derived substance is not limited to the protein. The amino acid-derived substance may be a peptide, a nucleic acid, or the like. Therefore, the pharmaceutical product is not limited to the biopharmaceutical product 11 that requires a biotechnology such as a recombinant DNA technology and a cell culture technology, and may be a peptide pharmaceutical product, a nucleic acid pharmaceutical product, or the like that can be manufactured only by a chemical synthesis technology without requiring the biotechnology.

The pharmaceutical product development support device 12 may be installed in the pharmaceutical facility or may be installed in a data center independent of the pharmaceutical facility.

Instead of delivering the screen data of the prediction result display screen 85 including the target formulation information 20T to the user terminal 13, the target formulation information 20T itself may be delivered to the user terminal 13. In this case, in the user terminal 13, the prediction result display screen 85 is generated based on the target formulation information 20T under the control of the browser control unit 77, and the prediction result display screen 85 is displayed on the display 29B.

A method of presenting the target formulation information 20T to the user U is not limited to the presentation by the delivery of the screen data described as an example. The target formulation information 20T may be presented to the user U by printing the target formulation information 20T on a paper medium, or the target formulation information 20T may be presented to the user U by attaching the target formulation information 20T to an e-mail and transmitting the e-mail to the user terminal 13.

A hardware configuration of the computer constituting the pharmaceutical product development support device 12 according to the technology of the present disclosure can be variously modified. For example, the pharmaceutical product development support device 12 can be configured by a plurality of computers separated as hardware for the purpose of improving processing capacity and reliability. For example, the functions of the request receiving unit 40 and the RW control unit 41 and the functions of the conversion unit 42, the derivation unit 43, and the screen distribution control unit 44 are distributed to two computers. In this case, the pharmaceutical product development support device 12 is configured by the two computers. In addition, a part or all of the functions of the pharmaceutical product development support device 12 may be performed by the user terminal 13.

As described above, the hardware configuration of the computer of the pharmaceutical product development support device 12 can be appropriately changed according to required performance such as processing capacity, safety, and reliability. Further, not only the hardware but also the AP such as the operation program 35, for the purpose of ensuring safety and reliability, may be duplicated or stored in a plurality of storage devices in a distributed manner.

In each of the embodiments, for example, a hardware structure of a processing unit that executes various types of processing, such as the request receiving unit 40, the RW control unit 41, the conversion unit 42, the derivation unit 43, the screen distribution control unit 44, and the browser control unit 77, can use the following various processors. The various processors include, in addition to the CPUs 27A and 27B that are general-purpose processors functioning as various processing units by executing software (the operation program 35 and the prediction AP 75) as described above, a programmable logic device (PLD) such as a field programmable gate array (FPGA) that is a processor having a circuit configuration changeable after manufacture, a dedicated electric circuit such as an application specific integrated circuit (ASIC) that is a processor having a circuit configuration dedicatedly designed to execute specific processing, and the like.

One processing unit may be configured by one of the various processors, or may be configured by a combination of two or more processors of the same type or different types (for example, a combination of a plurality of FPGAs and/or a combination of a CPU and an FPGA). Alternatively, a plurality of processing units may be composed of one processor.

As an example in which the plurality of processing units are configured by one processor, first, as represented by a computer such as a client and a server, there is a form in which one processor is configured by a combination of one or more CPUs and software and the processor functions as the plurality of processing units. Second, as represented by a system on chip (SoC) or the like, there is a form in which a processor that realizes the functions of the entire system including a plurality of processing units by one integrated circuit (IC) chip is used. As described above, as a hardware structure, various types of processing units are configured by using one or more of the various processors.

Further, as a hardware structure of the various processors, more specifically, an electrical circuit (circuitry) in which circuit elements such as semiconductor elements are combined can be used.

The technology according to the following supplementary notes can be understood from the above description.

### [Supplementary Note 1]

A pharmaceutical product development support device comprising a processor,
wherein the processor is configured to:
acquire target sequence information representing a sequence of amino acids constituting a substance that is amino acid-derived and contained in a target pharmaceutical product;
convert the target sequence information into target vector data reflecting a three-dimensional structure of the amino acids;
derive target formulation information representing a formulation of a storage solution suitable for the target pharmaceutical product based on the target vector data; and
present the target formulation information to a user.

### [Supplementary Note 2]

The pharmaceutical product development support device according to Supplementary Note 1,
wherein the processor is configured to:
convert the target sequence information into the target vector data by using a language model that treats the target sequence information as a sentence in natural language processing.

### [Supplementary Note 3]

The pharmaceutical product development support device according to Supplementary Note 1 or 2,
wherein the target formulation information is vector data representing a type of a buffer solution that is a base of the storage solution suitable for the target pharmaceutical product, and presence or absence of a salt, a sugar, an amino acid, and a surfactant contained in the storage solution suitable for the target pharmaceutical product.

### [Supplementary Note 4]

The pharmaceutical product development support device according to any one of Supplementary Notes 1 to 3,
wherein the processor is configured to:
derive the target formulation information by using a machine learning model that is trained by using training data composed of a set of reference vector data converted from reference sequence information representing a sequence of amino acids constituting a substance that is amino acid-derived and contained in a reference pharmaceutical product different from the target pharmaceutical product, and reference formulation information representing a formulation of a storage solution suitable for the reference pharmaceutical product.

### [Supplementary Note 5]

The pharmaceutical product development support device according to Supplementary Note 4,
wherein the reference formulation information is clustered into one of a plurality of clusters,
the machine learning model outputs one cluster of the plurality of clusters in response to input of the target vector data, and
the processor is configured to:
   derive, as the target formulation information, the reference formulation information belonging to the cluster output by the machine learning model.

### [Supplementary Note 6]

The pharmaceutical product development support device according to Supplementary Note 5,
wherein the processor is configured to:
derive the target formulation information based on a frequency of appearance of the reference formulation information belonging to the cluster output by the machine learning model.

### [Supplementary Note 7]

The pharmaceutical product development support device according to Supplementary Note 5,
wherein the processor is configured to:
receive designation of a derivation condition of the target formulation information by the user, and
derive the target formulation information based on the derivation condition.

### [Supplementary Note 8]

The pharmaceutical product development support device according to Supplementary Note 7,
wherein the derivation condition is the number of pieces of the target formulation information to be derived.

### [Supplementary Note 9]

The pharmaceutical product development support device according to Supplementary Note 7 or 8,
wherein the derivation condition is at least one of a type of a buffer solution that is a base of the storage solution or presence or absence of an additive contained in the storage solution.

### [Supplementary Note 10]

The pharmaceutical product development support device according to any one of Supplementary Notes 4 to 9,
wherein the reference formulation information is vector data representing a type of a buffer solution that is a base of the storage solution suitable for the reference pharmaceutical product, and presence or absence of a salt, a sugar, an amino acid, and a surfactant contained in the storage solution suitable for the reference pharmaceutical product.

### [Supplementary Note 11]

The pharmaceutical product development support device according to any one of Supplementary Notes 1 to 10,
wherein the substance is a protein.

### [Supplementary Note 12]

The pharmaceutical product development support device according to Supplementary Note 11,
wherein the protein is an antibody.

The technology of the present disclosure can also appropriately combine various embodiments and/or various modification examples described above. The technology of the present disclosure is not limited to the above embodiment and may adopt various configurations without departing from its gist. Furthermore, the technology of the present disclosure extends to a storage medium that non-transitorily stores the program, and a computer program product including the program, in addition to the program.

The description content and the shown content described above are detailed descriptions of parts related to the technology of the present disclosure and are merely an example of the technology of the present disclosure. For example, the above description of the configuration, the function, the action, and the effect are the description of examples of the configuration, the function, the action, and the effect of the parts according to the technology of the present disclosure. Accordingly, it goes without saying that unnecessary parts may be deleted, new elements may be added, or replacements may be made with respect to the description content and the shown content described above within a range that does not depart from the gist of the technology of the present disclosure. Further, in order to avoid complication and facilitate understanding of the parts according to the technology of the present disclosure, the description related to common general knowledge not requiring special description in order to implement the technology of the present disclosure is omitted in the description content and the shown content described above.

In the present specification, "A and/or B" is synonymous with "at least one of A or B". That is, "A and/or B" means that it may be only A, only B, or a combination of A and B. In addition, in the present specification, in a case where three or more matters are expressed by being connected by "and/or", the same concept as "A and/or B" is applied.

All documents, patent applications, and technical standards described in the present specification are incorporated in the present specification by reference to the same extent as in a case where each of the documents, the patent applications, and the technical standards are specifically and individually indicated to be incorporated by reference.

## Claims

1. A pharmaceutical product development support device comprising a processor,
wherein the processor is configured to:
acquire target sequence information representing a sequence of amino acids constituting a substance that is amino acid-derived and contained in a target pharmaceutical product;
convert the target sequence information into target vector data reflecting a three-dimensional structure of the amino acids;
derive target formulation information representing a formulation of a storage solution suitable for the target pharmaceutical product based on the target vector data; and
present the target formulation information to a user.

2. The pharmaceutical product development support device according to claim 1,
wherein the processor is configured to:
convert the target sequence information into the target vector data by using a language model that treats the target sequence information as a sentence in natural language processing.

3. The pharmaceutical product development support device according to claim 1,
wherein the target formulation information is vector data representing a type of a buffer solution that is a base of the storage solution suitable for the target pharmaceutical product, and presence or absence of a salt, a sugar, an amino acid, and a surfactant contained in the storage solution suitable for the target pharmaceutical product.

4. The pharmaceutical product development support device according to claim 1,
wherein the processor is configured to:
derive the target formulation information by using a machine learning model that is trained by using training data composed of a set of reference vector data converted from reference sequence information representing a sequence of amino acids constituting a substance that is amino acid-derived and contained in a reference pharmaceutical product different from the target pharmaceutical product, and reference formulation information representing a formulation of a storage solution suitable for the reference pharmaceutical product.

5. The pharmaceutical product development support device according to claim 4,
wherein the reference formulation information is clustered into one of a plurality of clusters,
the machine learning model outputs one cluster of the plurality of clusters in response to input of the target vector data, and
the processor is configured to:
derive, as the target formulation information, the reference formulation information belonging to the cluster output by the machine learning model.

6. The pharmaceutical product development support device according to claim 5,
wherein the processor is configured to:
derive the target formulation information based on a frequency of appearance of the reference formulation information belonging to the cluster output by the machine learning model.

7. The pharmaceutical product development support device according to claim 5,
wherein the processor is configured to:
receive designation of a derivation condition of the target formulation information by the user, and
derive the target formulation information based on the derivation condition.

8. The pharmaceutical product development support device according to claim 7,
wherein the derivation condition is the number of pieces of the target formulation information to be derived.

9. The pharmaceutical product development support device according to claim 7,
wherein the derivation condition is at least one of a type of a buffer solution that is a base of the storage solution or presence or absence of an additive contained in the storage solution.

10. The pharmaceutical product development support device according to claim 4,
wherein the reference formulation information is vector data representing a type of a buffer solution that is a base of the storage solution suitable for the reference pharmaceutical product, and presence or absence of a salt, a sugar, an amino acid, and a surfactant contained in the storage solution suitable for the reference pharmaceutical product.

11. The pharmaceutical product development support device according to claim 1,
wherein the substance is a protein.

12. The pharmaceutical product development support device according to claim 11,
wherein the protein is an antibody.

13. An operation method of a pharmaceutical product development support device, the operation method comprising:
acquiring target sequence information representing a sequence of amino acids constituting a substance that is amino acid-derived and contained in a target pharmaceutical product;
converting the target sequence information into target vector data reflecting a three-dimensional structure of the amino acids;
deriving target formulation information representing a formulation of a storage solution suitable for the target pharmaceutical product based on the target vector data; and
presenting the target formulation information to a user.

14. An operation program of a pharmaceutical product development support device, the operation program causing a computer to execute a process comprising:
acquiring target sequence information representing a sequence of amino acids constituting a substance that is amino acid-derived and contained in a target pharmaceutical product;
converting the target sequence information into target vector data reflecting a three-dimensional structure of the amino acids;
deriving target formulation information representing a formulation of a storage solution suitable for the target pharmaceutical product based on the target vector data; and
presenting the target formulation information to a user.
